# DEMANDE DE BREVET EUROPEEN

(11) **EP 1 323 753 A1**
(43) Date de publication de la demande: **02.07.2003**
(21) Numéro de dépôt: 02293169.5
(22) Date de dépôt: 19.12.2002
(51) Int. Cl.: C08F 220/34, C08F 220/60, A61K 7/06

(54) **Polymères radicalaires cationiques ou amphotères auto-adhésifs et leur utilisation en cosmétique**

(30) Priorité: 20.12.2001 FR 0116597
(71) Demandeur: L'OREAL, 75008 Paris (FR)
(72) Inventeur: Perron, Béatrice, 78350 Jouy en Josas (FR); Resile, Serge, 95390 Saint Prix (FR); Mougin, Nathalie, 75011 Paris (FR)
(74) Mandataire: Casalonga, Axel

(57) **Abrégé**

L'invention concerne des polymères radicalaires cationiques ou amphotères, comprenant un ou plusieurs motifs dérivés de monomères éthyléniques, cationiques ou ampholytiques, comportant au moins une fonction amine tertiaire ou quaternaire, caractérisés par le fait qu'ils présentent une auto-adhésivité - exprimée par la force de traction maximale (Fₘₐₓ (en N)) enregistrée lors du décollement par traction de deux surfaces circulaires de 0,95 cm², enduites de polymère - supérieure ou égale à 2 N, ainsi que leur utilisation en cosmétique, en particulier dans le domaine du coiffage.

## Description

La présente invention concerne de nouveaux polymères cationiques ou amphotères auto-adhésifs particuliers, obtenus par polymérisation radicalaire, l'utilisation de ces polymères auto-adhésifs en cosmétique, ainsi que des compositions cosmétiques, et en particulier des compositions de coiffage, les contenant.

Les polymères cationiques hydrosolubles, tels que les polymères à base de chlorure de diméthyldiallylammonium, sont utilisés depuis longtemps dans le domaine cosmétique, et en particulier dans le domaine des soins capillaires. En effet, leur bonne affinité (substantivité) pour les substrats kératiniques et en particulier leur capacité à former des films continus autour des cheveux en font d'excellents candidats pour protéger, embellir et renforcer les cheveux ou encore pour aider à déposer et à fixer d'autres substances sur les fibres kératiniques.

Cependant, en raison de leur viscosité élevée et de leur incompatibilité avec la plupart des agents propulseurs, les polymères de cette famille sont difficiles à utiliser dans des produits aérosol tels que des laques.

Les polymères cationiques couramment utilisés dans le domaine des soins capillaires présentent par ailleurs une faible auto-adhésivité, c'est-à-dire les fibres capillaires entourées d'une gaine de ces polymères cationiques n'adhèrent que peu ou pas du tout les unes aux autres.

La demanderesse a découvert une nouvelle famille de polymères radicalaires particuliers, cationiques ou amphotères, présentant une auto-adhésivité élevée et qui possèdent une substantivité suffisante et un très bon pouvoir coiffant. Cette association de propriétés les rend particulièrement appropriés pour une utilisation dans des compositions de coiffage à rincer telles que des shampooings coiffants.

Bien entendu, leur utilisation dans. des produits coiffants non-rincés est également avantageuse car ces polymères cationiques ou amphotères auto-adhésifs peuvent alors être utilisés en des quantités nettement plus faibles que les polymères cationiques ou amphotères connus ou les polymères auto-adhésifs anioniques ou neutres. La possibilité d'utiliser les polymères de la présent invention en de faibles quantités facilite leur formulation et réduit la viscosité des compositions obtenues.

Les polymères radicalaires cationiques ou amphotères auto-adhésifs de la présente invention peuvent également être utilisés dans des domaines cosmétiques autres que celui du coiffage. Ainsi, l'introduction de faibles quantités de ces polymères dans la plupart des produits de maquillage assure une bonne adhérence des dépôts cosmétiques sur la peau et leur confère une bonne cohésivité et souplesse. Le maquillage ne craquèle pas et ne tire pas la peau des utilisateurs.

L'invention a par conséquent pour objet des polymères radicalaires, cationiques ou amphotères, présentant une autoadhésivité - exprimée par la force de traction maximale (Fₘₐₓ (en N)) enregistrée lors du décollement par traction de deux surfaces circulaires de 0,95 cm², enduites de polymère - supérieure à 2 N et comprenant un ou plusieurs motifs dérivés de monomères choisis parmi ceux de formule (la), (Ib), (Ic), (Id) et (le) 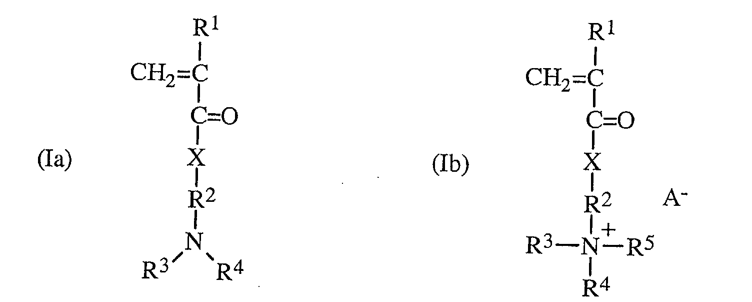 dans lesquelles
R₁ représente un atome d'hydrogène ou un groupe méthyle,
R₂ représente un groupe hydrocarboné divalent en C₁₋₃₀, linéaire, ramifié, cycloaliphatique ou aromatique, pouvant contenir un ou plusieurs hétéroatomes choisis parmi O, N et P,
R₃, R₄ et R₅ représentent chacun indépendamment un groupe hydrocarboné en C₁₋₃₀, linéaire, ramifié, cycloaliphatique ou aromatique, pouvant contenir un ou plusieurs hétéroatomes choisis parmi O, N et P,
X représente un atome d'oxygène ou un groupe NH,
A⁻ représente le contre-ion de l'amine quaternaire, choisi de préférence parmi les ions halogénure, sulfate, phosphate et carboxylate tel qu'acétate, 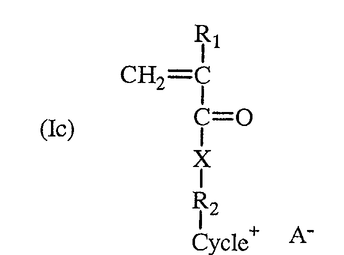
dans laquelle
X, R₁ et R₂ ont la signification indiquée à propos des formules (la) et (Ib), et
Cycle⁺ représente un système monocyclique ou bicyclique condensé, cycloaliphatique ou aromatique, comportant une fonction amine tertiaire ou quaternaire, et pouvant renfermer un ou plusieurs hétéroatomes supplémentaires choisis parmi O, N ou P ; 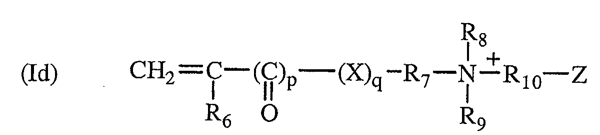
dans laquelle
R₆ représente un atome d'hydrogène ou un groupe alkyle en C₁₋₄ linéaire ou ramifié,
R₈ et R₉ représentent chacun indépendamment un atome d'hydrogène ou un groupe alkyle en C₁₋₄ linéaire ou ramifié, portant éventuellement un groupe COO⁻, SO₃⁻ ou PO₃H⁻,
R₇ et R₁₀ représentent chacun indépendamment un groupe hydrocarboné divalent, en particulier un groupe -(CH₂)ₙ avec n compris entre 1 et 4 inclus, et éventuellement interrompu par un atome d'oxygène,
X est un atome d'oxygène ou un groupe NH,
p et q valent 0 ou 1
Z représente un groupe COO⁻, SO₃⁻ ou PO₃H⁻,
où R₇ peut former avec R₈, R₉ ou X, lorsque ce dernier représente un groupe NH, un hétérocycle à 5, 6 ou 7 chaînons, aromatique ou non-aromatique ; 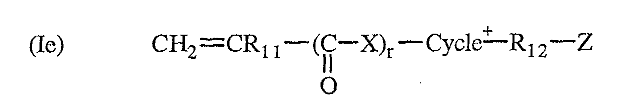
dans laquelle
R₁₁ représente un atome d'hydrogène ou un groupe méthyle,
R₁₂ représente un groupe hydrocarboné divalent en C₁₋₄,
X représente un atome d'oxygène ou un groupe NH,
r vaut 0 ou 1,
Cycle⁺ représente un système monocyclique ou bicyclique condensé, cycloaliphatique ou aromatique, comportant une fonction amine tertiaire ou quaternaire, et pouvant renfermer un ou plusieurs hétéroatomes supplémentaires choisis parmi O, N ou P, et
Z représente un groupe COO⁻, SO₃⁻ ou PO₃H⁻.

L'invention a également pour objet une composition cosmétique, et en particulier une composition de coiffage, contenant, dans un milieu cosmétiquement acceptable, au moins un tel polymère radicalaire cationique ou amphotère auto-adhésif.

L'invention a enfin pour objet l'utilisation en cosmétique des nouveaux polymères radicalaires cationiques ou amphotères auto-adhésifs décrits ci-dessus, et en particulier un procédé de traitement des matières kératiniques et un procédé de coiffage utilisant ces polymères.

Le caractère auto-adhésif des polymères cationiques ou amphotères de la présente invention est évalué selon le protocole suivant :

On dépose sur la surface de deux plaquettes circulaires en verre dépoli, ayant chacune une surface de 0,95 cm² (diamètre 11 mm), 40 µl d'une solution ou dispersion aqueuse contenant 10 % en poids de polymère à tester. On laisse sécher pendant 48 heures à pression ambiante, à un taux d'humidité relative de 55 % et à une température de 22 °C.

Les deux plaquettes sont fixées dans un appareil de mesure de la résistance en traction (Lloyd LR5K) et sont pressées l'une contre l'autre pendant 20 secondes avec une force de 3 N. On sépare ensuite, dans les mêmes conditions de température et d'humidité relative, les deux plaquettes pendant 30 secondes en imposant une vitesse de traction de 20 mm/minute, et l'on enregistre la force nécessaire à ce déplacement, et plus particulièrement la force maximale (Fₘₐₓ) en Newton (N) mesurée au moment de la séparation brusque des deux surfaces revêtues de polymère. Bien entendu, l'auto-adhésivité des polymères de la présente invention est d'autant plus forte que la force maximale enregistrée est élevée.

Les polymères radicalaires auto-adhésifs cationiques ou amphotères de la présente invention une auto-adhésivité telle que Fₘₐₓ est supérieure ou égale à 2 N, de préférence comprise entre 2 et 100 et en particulier entre 5 N et 100 N.

Les polymères radicalaires cationiques ou amphotères autoadhésifs de la présente invention ont de préférence une température de transition vitreuse (Tg) inférieure à la température ambiante (20 °C), c'est-à-dire à température ambiante, il sont à l'état plastique et non à l'état vitreux. Les caractéristiques d'auto-adhésivité des polymères de la présente invention sont particulièrement intéressantes lorsque la température de transition vitreuse est inférieure à 0 °C et en particulier inférieure à -20 °C.

Lorsque les polymères radicalaires cationiques ou amphotères de la présente invention présentent plusieurs températures de transition vitreuse, la Tg la plus basse est de préférence inférieure à 20 °C, en particulier inférieure à 0 °C et idéalement inférieure à -20 °C.

La température de transition vitreuse des polymères de la présente invention est mesurée par analyse enthalpique différentielle (en anglais *Differential Scanning Calorimetry*, DSC) dans les conditions suivantes :

Pour mesurer la température de transition vitreuse, on réalise un film ayant une épaisseur d'environ 150 mm du polymère à tester en déposant une solution ou une dispersion aqueuse du polymère dans une matrice circulaire en téflon de 40 mm de diamètre et en laissant sécher le dépôt. Le film est mis à sécher dans une étuve à une température d'environ 23 °C sous une humidité realtive de 45 %, jusqu'à ce que le poids ne varie plus. On prélève environ 5 à 15 mg du film, que l'on place dans un creuset qui est introduit ensuite dans l'analyseur. L'analyseur thermique est un modèle DSC-2920 de la société TA INSTRUMENTS. Les températures initiales et finales du balayage en température sont choisies de manière à encadrer la température de transition vitreuse recherchée. Le balayage en température se fait à une vitesse de 10 °C/minute.

Cette analyse est effectuée selon la norme ASTM D 3418-97 aux modifications ci-dessus près.

On peut citer à titre d'exemples de monomères éthyléniques cationiques définis par les formules (la) à (Ic) ci-dessus le (méth)acrylate de diméthylaminoéthyle, le (méth)acrylate de diéthylaminoéthyle, le (méth)acrylate de diméthylaminopropyle, le diméthylaminoéthyl(méth)-acrylamide, le diméthylaminopropyl(méth)-acrylamide, le (méth)acrylate de N-morpholinoéthyle, le chlorure de (méth)acrylate de triméthylammonio-éthyle, le chlorure de (méth)acrylate de triméthylammoniopropyle, le chlorure de triméthylammonioéthyl(méth)acrylamide, le chlorure de triméthyl-ammoniopropyl(méth)acrylamide et le chlorure de (méth)acrylate de diméthylbenzylammonioéthyle.

On peut citer à titre d'exemples de monomères amphotères de formule (Id) ou (Ie) particulièrement préférés l'hydroxyde de 1-vinyl-2-(3-sulfopropyl)imidazolium, le 1-vinyl-3-(3-sulfopropyl)imidazolium, l'hydroxyde de 1-vinyl-3-(4-sulfobutyl)imidazolium, l'hydroxyde de 1-vinyl-2-méthyl-3-(4-sulfobutyl)imidazolium, l'hydroxyde de 2-vinyl-1-(3-sulfopropyl)pyridinium, l'hydroxyde de 2-méthyl-5-vinyl-1-(3-sulfopropyl)-pyridinium, l'hydroxyde de 4-vinyl-1-(3-sulfopropyl)pyridinium, l'hydroxyde de diméthyl-(2-méthacryloxyéthyl)-3-sulfopropyl)ammonium, l'hydroxyde de diéthyl-(2-méthacryloxyéthoxy)-2-éthyl-(3-sulfopropyl)ammonium, le 4-vinyl-4-(sulfobutyl)pyridinium, l'hydroxyde de N-(3-sulfopropyl)-N-méthacrylamidopropyl-N,N-diméthylammonium, l'hydroxyde de N,N-diméthyl-N-(3-(méthacrylamido)propyl)-3-(sulfopropyl)ammonium, l'hydroxyde de N,N-diméthyl-N-(3-méthacrylamidopropyl)-N-(3-carboxy-propyl)ammonium et l'hydroxyde de N,N-diméthyl-N-(2-méthacryloxyéthyl-N-(3-carboxypropyl)ammonium.

Des comonomères cationiques tout particulièrement préférés selon l'invention sont le méthacrylate de diméthylaminoéthyle (MADAME) et le diméthylaminopropylméthacrylamide (DMAPMA).

Les polymères cationiques ou amphotères auto-adhésifs de la présente invention comprennent de préférence - outre les motifs dérivés de monomères cationiques et/ou amphotères - des motifs dérivés de monomères éthyléniques non-ioniques.

La demanderesse a constaté que les propriétés d'auto-adhésivité des polymères de la présente invention étaient particulièrement intéressantes, lorsque ces monomères non-ioniques étaient choisis parmi ceux qui forment, lorsqu'on les homopolymérisent, des polymères ayant une température de transition vitreuse inférieure à 0 °C.

Ces comonomères non-ioniques sont connus dans la technique et peuvent être décrits par les formules (IIa) ou (IIb) : 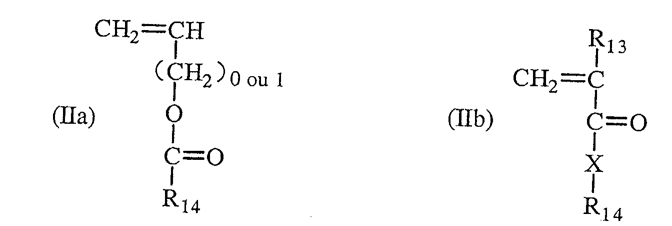 dans lesquelles
R₁₃ représente un atome d'hydrogène ou un groupe méthyle,
X représente un atome d'oxygène ou un groupe NH, et
R₁₄ représente un groupe hydrocarboné en C₂₋₆₀, linéaire, ramifié, cycloaliphatique ou aromatique, pouvant contenir un ou plusieurs atomes choisis parmi O, S et P, et notamment une chaîne alcoxy-polyéthylèneglycol.

Parmi ces comonomères non-ioniques donnant éventuellement des homopolymères à Tg < 0 °C, on préfère en particulier l'acrylate d'éthyle, de n-butyle, de n-hexyle, de 2-éthylhexyle, de n-nonyle, de lauryle, de n-octadécyle, d'iso-octyle, d'isodécyle, d'hydroxyéthyle, d'hydroxypropyle et de méthoxyéthyle, le méthacrylate de n-hexyle, de 2-éthylhexyle, d'éthoxyéthyle, d'isodécyle, de méthoxyéthyle, d'alcoxy en C₁₋₃₀-PEG (avec 5 à 30 motifs d'oxyde d'éthylène), le propionate de vinyle, et les néoalcanoates de vinyle tels que le néononanoate de vinyle et le néododécanoate de vinyle.

Des comonomères particulièrement préférés de ce groupe sont l'acrylate de n-butyle, le méthacrylate d'éthoxyéthyle, l'acrylate de 2-éthylhexyle et le méthacrylate de méthoxyPEG (avec 5 à 30 motifs d'oxyde d'éthylène).

Les polymères autoadhésifs de la présente invention peuvent également contenir une faible quantité de monomères non-ioniques fortement hydrophobes tels que les monomères vinyliques à chaîne latérale siliconée, le chlorotrifluoroéthylène, le tétrafluoroéthylène, et les monomères vinyliques, allyliques ou (méth)acryliques à chaîne latérale hydrocarbonée perhalogénée, en particulier perfluorée, tels que le (méth)acrylate de perfluorohexyle ou le (méth)acrylate de perfluorooctyle.

Lorsque les polymères cationiques ou amphotères auto-adhésifs de la présente invention sont constitués à la fois de monomères cationiques ou amphotères et de monomères non-ioniques donnant des homopolymères à Tg < 0°C, les motifs dérivés de monomères éthyléniques cationiques ou amphotères, représentent de 1 à 50 % en poids du polymère, de préférence de 1 à 20 % en poids du polymère, et les motifs dérivés de monomères éthyléniques non-ioniques donnant des homopolymères à Tg < 0 °C représentent de 50 % à 99 % en poids du polymère, de préférence de 80 à 99 % en poids du polymère.

Les polymères autoadhésifs de la présente invention peuvent comporter - en plus des motifs dérivés de monomères cationiques ou amphotères et des motifs dérivés de monomères non-ioniques donnant des homopolymères à Tg < 0 °C - une certaine proportion de comonomères éthyléniques anioniques.

Ces comonomères sont des monomères éthyléniques portant au moins une fonction acide carboxylique, acide sulfonique ou acide phosphonique.

Ils sont choisis par exemple parmi l'acide acrylique, l'acide méthacrylique, l'acide itaconique, l'acide crotonique, l'acide fumarique, l'acide maléique, l'acide vinylbenzoïque, l'acide vinylbenzènesulfonique, l'acide acrylamidopropanesulfonique et l'acide vinylphosphonique ou parmi les sels d'addition de bases minérales ou organiques de ces acides.

L'introduction de ces comonomères anioniques permet d'ajuster l'équilibre des charges, de modifier le caractère hydrophile et donc la solubilité des polymères obtenus ou encore de moduler la compatibilité des polymères avec certains substrats ou supports cosmétiques.

Pour garantir une bonne affinité des polymères auto-adhésifs de la présente invention pour les substrats cosmétiques, et en particulier les fibres kératiniques, il est généralement souhaitable que la charge globale des polymères amphotères soit positive, c'est-à-dire que le nombre total de charges positives portées par les polymères soit supérieur au nombre de charges négatives.

Parmi les copolymères radicalaires cationiques ou amphotères décrits ci-dessus, la demanderesse a obtenu des résultats particulièrement intéressants avec les polymères suivants :
- les copolymères d'acrylate de butyle et de méthacrylate de diméthylaminoéthyle,
- les copolymères d'acrylate de butyle et de diméthylaminopropylméthacrylamide,
- les terpolymères de méthacrylate méthoxy-PEG(avec de 5 à 30 motifs OE), de diméthylaminopropylméthacrylamide et de méthacrylate d'éthoxyéthyle, et
- les terpolymères d'acrylate de 2-éthylhexyle, de diméthylaminopropylméthacrylamide et de méthacrylate d'éthoxyéthyle.

Bien que la masse moléculaire des polymères cationiques ou amphotères auto-adhésifs de la présente invention ne soit pas un facteur déterminant pour la présente invention, on utilise de préférence des polymères ayant une masse molaire moyenne en nombre comprise entre 5000 et 5 000 000, de préférence comprise entre 50 000 et 5 000 000.

Les polymères cationiques ou amphotères auto-adhésifs de la présente invention peuvent être solubles ou insolubles dans l'eau. Lorsqu'ils sont insolubles dans l'eau, ils se présentent le plus souvent sous forme de latex, c'est-à-dire sous forme de dispersions de fines particules, ayant une taille moyenne comprise entre 3 nm et 600 nm, de préférence entre 5 nm et 400 nm, dans une phase aqueuse.

Les formes dispersées, insolubles dans l'eau des polymères de la présente invention sont préférées car elles ne présentent pas les problèmes de viscosité élevée des solutions et sont plus faciles à manipuler que celles-ci.

Les polymères cationiques ou amphotères auto-adhésifs de la présente invention peuvent être préparés par polymérisation radicalaire en solution, en masse, en dispersion ou en émulsion, familières à l'homme du métier.

Les charges positives des polymères peuvent être introduites par copolymérisation de monomères à fonction amine tertiaire protonée ou à fonction amine quaternaire, mais la protonation ou la quaternisation des fonctions amine peut également se faire après polymérisation.

Les agents de protonation des fonctions amine des polymères auto-adhésifs de la présente invention sont choisis parmi les acides organiques ou minéraux cosmétiquement acceptables tels que l'acide chlorhydrique, l'acide acétique, l'acide glycolique et l'acide succinique.

Les agents d'alkylation sont des composés connus possédant une ou plusieurs chaînes alkyle et un groupe partant approprié tels qu'un atome d'halogène ou un groupe sulfate. On peut citer à titre d'exemples les halogénures d'alkyle en C₁-C₃₀ tels que le chlorure de méthyle, et les sulfates de dialkyle comme le sulfate de diéthyle.

Comme indiqué ci-dessus, les polymères cationiques ou amphotères autoadhésifs de la présente invention peuvent être utilisés en cosmétique sous forme de compositions de soin ou de maquillage de la peau ou des phanères, en particulier sous forme de compositions de soin, de maquillage ou de fixation des matières kératiniques humaines telles que les cheveux et des cils.

De préférence, ces compositions cosmétiques contiennent, dans un milieu aqueux cosmétiquement acceptable, de 0,01 % à 40 %, en particulier de 0,05 à 20 % et idéalement de 0,1 à 10 % en poids d'au moins un polymère cationique ou amphotère auto-adhésif de la présente invention.

Dans un mode de réalisation préféré de la présent invention, les compositions cosmétiques sont des compositions de coiffage, et en particulier des compositions de coiffage à rincer, à savoir des shampooings coiffants.

Le milieu aqueux cosmétiquement acceptable peut contenir différents adjuvants et solvants couramment utilisés dans le domaine cosmétique tels que des agents tensioactifs, des polymères anioniques, amphotères, zwitterioniques ou non ioniques, des polymères cationiques différents des polymères auto-adhésifs cationiques de la présente invention, des agents nacrants et/ou opacifiants, des solvants organiques, des parfums, des huiles minérales, végétales et/ou synthétiques, des esters d'acides gras, des pigments et colorants, des silicones, des particules minérales ou organiques, des agents de stabilisation du pH, des agents conservateurs et des agents absorbant les UV.

Parmi les polymères cationiques susceptibles d'être utilisés dans les compositions de la présente invention, on préfère les dérivés d'éther de cellulose quaternaires tels que les produits commercialisés sous la dénomination "JR 400" par la société UNION CARBIDE CORPORATION, les cyclopolymères, en particulier les homopolymères de sel de diallyldiméthylammonium et les copolymères de sel de diallyldiméthylammonium et d'acrylamide en particulier les chlorures, commercialisés sous les dénominations "MERQUAT 100", "MERQUAT 550" et "MERQUAT S" par la société MERCK, les polysaccharides cationiques et plus particulièrement les gommes de guar modifiées par du chlorure de 2,3-époxypropyl triméthylammonium commercialisées par exemple sous la dénomination "JAGUAR C13S" par la société MEYHALL, les homopolymères et les copolymères éventuellement réticulés de sel de (méth)acryloyloxyéthyltriméthylammonium, vendus par la société ALLIED COLLOIDS en solution à 50% dans de l'huile minérale sous les dénominations commerciales SALCARE SC92 (copolymère réticulé du chlorure de méthacryloyloxyéthyltriméthylammonium et de l'acrylamide) et SALCARE SC95 (homopolymère réticulé du chlorure de méthacryloyloxyéthyl triméthylammonium).

On peut également utiliser les polymères qui sont constitués de motifs récurrents répondant à la formule : 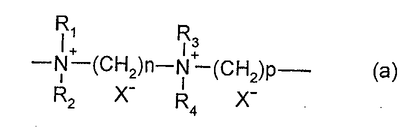 dans laquelle R₁, R₂, R₃ et R₄, identiques ou différents, désignent un radical alkyle ou hydroxyalkyle ayant de 1 à 4 atomes de carbone environ, n et p sont des nombres entiers variant de 2 à 20 environ et, X⁻ est un anion dérivé d'un acide minéral ou organique.

Les agents tensioactifs utilisables dans la composition selon la présente invention peuvent être des agents tensioactifs anioniques, non ioniques, amphotères ou cationiques, ou des mélanges de ceux-ci.

Parmi les agents tensioactifs anioniques utilisables, seuls ou en mélanges, dans le cadre de la présente invention, on peut notamment citer les sels, et en particulier les sels de métaux alcalins tels que les sels de sodium, les sels d'ammonium, les sels d'amines, les sels d'aminoalcools ou les sels de métaux alcalino-terreux, par exemple, de magnésium, des composés suivants : les alkylsulfates, les alkyléthersulfates, les alkylamidoéthersulfates, les alkylarylpolyéthersulfates, les monoglycéride-sulfates ; les alkylsulfonates, les alkylamidesulfonates, les alkyl-arylsulfonates, les α-oléfine-sulfonates, les paraffine-sulfonates; les alkylsulfosuccinates, les alkyléthersulfosuccinates, les alkylamide-sulfosuccinates ; les alkylsulfoacétates ; les acylsarcosinates ; et les acylglutamates, les groupes alkyle et acyle de tous ces composés comportant de 6 à 24 atomes de carbone et le groupe aryle désignant de préférence un groupe phényle ou benzyle.

On peut également utiliser dans le cadre de la présente invention les esters d'alkyle en C₆-C₂₄ et d'acides polyglycoside-carboxyliques tels que les glucoside-citrates d'alkyle, les polyglycoside-tartrates d'alkyle, et les polyglycoside-sulfosuccinates d'alkyle ; les alkylsulfosuccinamates, les acyliséthionates et les N-acyltaurates, le groupe alkyle ou acyle de tous ces composés comportant de 12 à 20 atomes de carbone. Parmi les tensioactifs anioniques encore utilisables, on peut également citer les acyllactylates dont le groupe acyle comporte de 8 à 20 atomes de carbone.

En outre, on peut encore citer les acides d'alkyl-D-galactoside uroniques et leurs sels ainsi que les acides alkyl(C₆-C₂₄)éther-carboxyliques polyoxyalkylénés, les acides alkyl(C₆-C₂₄)aryl(C₆-C₂₄)éther-carboxyliques polyoxyalkylénés, les acides alkyl(C₆-C₂₄)amidoéther carboxyliques polyoxyalkylénés et leurs sels, en particulier ceux comportant de 2 à 50 groupements oxyde d'éthylène, et leurs mélanges.

Parmi les tensioactifs anioniques cités ci-dessus, on préfère utiliser selon l'invention les alkyl(C₆-C₂₄)sulfates, les alkyl(C₆-C₂₄)éthersulfates, les alkyl(C₆-C₂₄)éthercarboxylates et leurs mélanges, par exemple le laurylsulfate d'ammonium, le laurylsulfate de sodium, le laurylsulfate de magnésium, le lauryléthersulfate de sodium, le lauryléthersulfate d'ammonium et le lauryléthersulfate de magnésium.

La composition selon la présente invention peut comprendre les agents tensioactifs anioniques en une quantité comprise de préférence entre 0,5 et 60 % en poids, mieux encore entre 5 et 20 % en poids par rapport au poids total de la composition.

Les agents tensioactifs non-ioniques que l'on peut utiliser dans le cadre de la présente invention, sont eux aussi, des composés bien connus en soi (voir notamment à cet égard "Handbook of Surfactants" par M.R. PORTER, éditions Blackie & Son (Glasgow and London), 1991, pp 116-178). Ils peuvent être notamment choisis parmi les alcools, les alpha-diols, les alkyl(C₁-C₂₀)phénols ou les acides gras polyéthoxylés, polypropoxylés ou polyglycérolés, ayant une chaîne grasse comportant, par exemple, de 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et d'oxyde de propylène, les condensats d'oxyde d'éthylène et d'oxyde de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitane éthoxylés ayant de 2 à 30 moles d'oxyde d'éthylène ; les esters d'acides gras du saccharose, les esters d'acides gras du polyéthylèneglycol, les alkyl(C₆-C₂₄)polyglucosides, les dérivés de N-alkyl(C₆-C₂₄)glucamine, les oxydes d'amines tels que les oxydes d'alkyl(C₁₀-C₁₄)amines ou les oxydes de N-acyl(C₁₀-C₁₄)aminopropylmorpholine ; et leurs mélanges.

Parmi les tensioactifs non-ioniques cités ci-dessus, on utilise de préférence les alkyl(C₆-C₂₄)polyglycosides, en particulier le décylpolyglucoside.

Les agents tensioactifs amphotères, convenant dans la présente invention, peuvent être notamment des dérivés d'amines aliphatiques secondaires ou tertiaires, dans lesquels le groupe aliphatique est une chaîne linéaire ou ramifiée comportant de 8 à 22 atomes de carbone et contenant, au moins un groupe anionique hydrosolubilisant tel que, par exemple, un groupe carboxylate, sulfonate, sulfate, phosphate ou phosphonate ; on peut citer encore les alkyl(C₈-C₂₀)bétaïnes, les sulfobétaïnes, les alkyl(C₈-C₂₀)amidoalkyl(C₆-C₈)-bétaïnes ou les alkyl(C₈-C₂₀)amidoalkyl(C₆-C₈)sulfobétaïnes ; et leurs mélanges.

Parmi les dérivés d'amines, on peut citer les produits commercialisés sous la dénomination MIRANOL®, tels que décrits dans les brevets US 2 528 378 et US 2 781 354 et classés dans le dictionnaire CTFA, 3ème édition, 1982, sous les dénominations Amphocarboxy-glycinate et Amphocarboxypropionate de structures respectives (1) et (2) :

R₂-CONHCH₂CH₂-N⁺(R₃)(R₄)(CH₂COO⁻) (1)

dans laquelle :
R₂ représente un groupe alkyle dérivé d'un acide R₂-COOH présent dans l'huile de coprah hydrolysée, un groupe heptyle, nonyle ou undécyle,
R₃ représente un groupe bêta-hydroxyéthyle, et
R₄ représente un groupe carboxyméthyle ;
   et

   R₂'-CONHCH₂CH₂-N(B)(C) (2)
dans laquelle :
B représente -CH₂CH₂OX',
C représente -(CH₂)_{z}-Y', avec z = 1 ou 2,
X' représente le groupe -CH₂CH₂-COOH ou un atome d'hydrogène,
Y' représente -COOH ou le groupe -CH₂-CHOH-SO₃H,
R₂' représente le groupe alkyle d'un acide R₂'-COOH présent dans l'huile de coprah ou dans l'huile de lin hydrolysée, un groupe alkyle, notamment en C₁₇ et sa forme iso, un groupe en C₁₇ insaturé.

Ces composés sont classés dans le dictionnaire CTFA, 5^{ème} édition, 1993, sous les dénominations cocoamphodiacétate de disodium, lauroamphodiacétate de disodium, caprylamphodiacétate de disodium, capryloamphodiacétate de disodium, cocoamphodipropionate de disodium, lauroamphodipropionate de disodium, caprylamphodipropionate de disodium, capryloamphodipropionate de disodium, acide lauroamphodipropionique, acide cocoamphodipropionique.

A titre d'exemple, on peut citer le cocoamphodiacétate commercialisé sous la dénomination commerciale MIRANOL® C2M concentré par la société RHODIA.

Parmi les tensioactifs amphotères, on utilise de préférence les alkyl(C₈-C₂₀)bétaïnes telles que la cocobétaïne, les alkyl(C₈-C₂₀)amidoalkyl(C₆-C₈)bétaïnes telles que la cocamidobétaïne, les alkylamphodiacétates comme le cocoamphodiacétate disodique, et leurs mélanges.

La composition selon l'invention peut comprendre en outre un ou plusieurs tensioactifs cationiques bien connus en soi, tels que les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhy droxyalkylammonium ou d'alkylpyridinium ; les dérivés d'imidazoline ; ou les oxydes d'amines à caractère cationique.

Les agents tensioactifs non ioniques, amphotères et cationiques décrits ci-dessus peuvent être utilisés seuls ou en mélanges et leur quantité est comprise entre 0,1 % et 30 % en poids, de préférence entre 0,5 % et 25 % en poids et mieux encore entre 1 % et 20 % en poids par rapport au poids total de la composition.

Les silicones utilisables en tant qu'additifs dans les compositions cosmétiques de la présente invention, sont des silicones volatiles ou non volatiles, cycliques, linéaires ou ramifiées, modifiées ou non par des groupements organiques, ayant une viscosité de 5.10⁻⁶ à 2,5 m²/s à 25°C et de préférence 1.10⁻⁵ à 1 m²/s.

Les silicones utilisables conformément à l'invention peuvent être solubles ou insolubles dans la composition et en particulier être des polyorganosiloxanes insolubles dans la composition de l'invention. Elles peuvent se présenter sous forme d'huiles, de cires, de résines ou de gommes.

Les organopolysiloxanes sont définis plus en détail dans l'ouvrage de Walter NOLL "Chemistry and Technology of Silicones" (1968), Academic Press. Elles peuvent être volatiles ou non volatiles.

Lorsqu'elles sont volatiles, les silicones sont plus particulièrement choisies parmi celles possédant un point d'ébullition compris entre 60° C et 260° C, et plus particulièrement encore parmi :
(i) les silicones cycliques comportant de 3 à 7, de préférence de 4 à 5 atomes de silicium. Il s'agit, par exemple, de l'octaméthylcyclotétrasiloxane commercialisé notamment sous le nom de VOLATILE SILICONE® 7207 par UNION CARBIDE ou SILBIONE® 70045 V 2 par RHODIA, le décaméthylcyclopentasiloxane commercialisé sous le nom de VOLATILE SILICONE® 7158 par UNION CARBIDE, et SILBIONE® 70045 V 5 par RHODIA, ainsi que leurs mélanges.

On peut également citer les cyclocopolymères du type diméthylsiloxane/méthylalkylsiloxane, tel que la SILICONE VOLATILE® FZ 3109 commercialisée par la société UNION CARBIDE, de formule :
avec D :

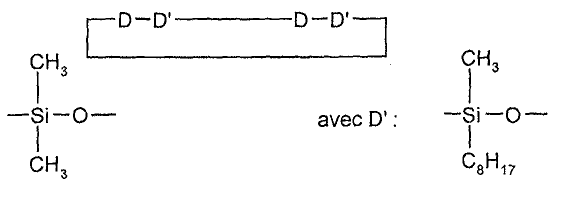

On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'-(hexa-2,2,2',2',3,3'- triméthylsilyloxy) bis-néopentane ;
(ii) les silicones volatiles linéaires ayant 2 à 9 atomes de silicium et présentant une viscosité inférieure ou égale à 5.10⁻⁶m²/s à 25 °C. Il s'agit, par exemple, du décaméthyltétrasiloxane commercialisé notamment sous la dénomination SH 200 par la société TORAY SILICONE. Des silicones entrant dans cette classe sont également décrites dans l'article publié dans *Cosmetics and Toiletries*, Vol. 91, Jan. 76, p. 27-32 - TODD & BYERS *"Volatile Silicone Fluids for Cosmetics".*

On utilise de préférence des silicones non volatiles et plus particulièrement des polyalkylsiloxanes, des polyarylsiloxanes, des polyalkylarylsiloxanes, des gommes et des résines de silicones, des polyorganosiloxanes modifiés par des groupements organofonctionnels ainsi que leurs mélanges.

Ces silicones sont plus particulièrement choisies parmi les polyalkylsiloxanes parmi lesquels on peut citer principalement les polydiméthylsiloxanes à groupements terminaux triméthylsilyle. La viscosité des silicones est mesurée à 25°C selon la norme ASTM 445 Appendice C.

Parmi ces polyalkylsiloxanes, on peut citer à titre non limitatif les produits commerciaux suivants :
- les huiles SILBIONE® des séries 47 et 70 047 ou les huiles MIRASIL® commercialisées par RHODIA telles que, par exemple, l'huile 70 047 V 500 000 ;
- les huiles de la série MIRASIL® commercialisées par la société RHODIA ;
- les huiles de la série 200 de la société DOW CORNING telles que la DC200 ayant une viscosité de 60 000 mm²/s ;
- les huiles VISCASIL® de GENERAL ELECTRIC et certaines huiles des séries SF (SF 96, SF 18) de GENERAL ELECTRIC.

On peut également citer les polydiméthylsiloxanes à groupements terminaux diméthylsilanol, connus sous le nom de dimethiconol (CTFA), tels que les huiles de la série 48 de la société RHODIA .

Dans cette classe de polyalkylsiloxanes, on peut également citer les produits commercialisés sous les dénominations ABIL WAX® 9800 et 9801 par la société GOLDSCHMIDT qui sont des polyalkyl(C₁-C₂₀)siloxanes.

Les polyalkylarylsiloxanes sont particulièrement choisis parmi les polydiméthyl/méthylphénylsiloxanes, les polydiméthyldiphényl-siloxanes linéaires et/ou ramifiés de viscosité allant de 1.10⁻⁵ à 5.10⁻² m²/s à 25°C.

Parmi ces polyalkylarylsiloxanes on peut citer, à titre d'exemple, les produits commercialisés sous les dénominations suivantes
- les huiles SILBIONE® de la série 70 641 de RHODIA ;
- les huiles des séries RHODORSIL® 70 633 et 763 de RHODIA ;
- l'huile DOW CORNING 556 COSMETIC GRAD FLUID de DOW CORNING ;
- les silicones de la série PK de BAYER comme le produit PK20 ;
- les silicones des séries PN, PH de BAYER comme les produits PN1000 et PH1000 ;
- certaines huiles des séries SF de GENERAL ELECTRIC telles que SF 1023, SF 1154, SF 1250, SF 1265.

Les gommes de silicone utilisables conformément à l'invention sont notamment des polydiorganosiloxanes ayant des masses moléculaires moyennes en nombre élevées comprises entre 200 000 et 1 000 000, utilisés seuls ou en mélange dans un solvant. Ce solvant peut être choisi parmi les silicones volatiles, les huiles polydiméthylsiloxanes (PDMS), les huiles polyphénylméthylsiloxanes (PPMS), les isoparaffines, les polyisobutylènes, le chlorure de méthylène, le pentane, le dodécane, le tridécane ou leurs mélanges.

On peut plus particulièrement citer les produits suivants :
- polydiméthylsiloxane
- les gommes polydiméthylsiloxane/méthylvinylsiloxane,
- polydiméthylsiloxane/diphénylsiloxane,
- polydiméthylsiloxane/phénylméthylsiloxane,
- polydiméthylsiloxane/diphénylsiloxane/méthylvinylsiloxane.

Des produits plus particulièrement utilisables conformément à l'invention sont des mélanges tels que :
- les mélanges formés à partir d'un polydiméthylsiloxane hydroxylé en bout de chaîne, ou diméthiconol (CTFA), et d'un polydiméthylsiloxane cyclique, également appelé cyclométhicone (CTFA), tel que le produit Q2 1401 commercialisé par la société DOW CORNING ;
- les mélanges d'une gomme polydiméthyl-siloxane et d'une silicone cyclique, tels que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10⁻⁶ m²/s. Ce produit comporte de préférence 15 % de gomme SE 30 et 85 % d'une huile SF 96.

Les résines d'organopolysiloxanes utilisables conformément à l'invention sont des systèmes siloxaniques réticulés renfermant les motifs :

R₂SiO_{2/2}, R₃SiO_{1/2}, RSiO_{3/2} et SiO_{4/2}

dans lesquels R représente un groupement hydrocarboné possédant 1 à 16 atomes de carbone ou un groupement phényle. Parmi ces produits, ceux particulièrement préférés sont ceux dans lesquels R désigne un groupe alkyle inférieur en C₁-C₄, plus particulièrement méthyle, ou un groupe phényle.

On peut citer parmi ces résines le produit commercialisé sous la dénomination DOW CORNING 593 ou ceux commercialisés sous les dénominations SILICONE FLUID SS 4230 et SS 4267 par la société GENERAL ELECTRIC et qui sont des silicones de structure diméthyl/triméthylsiloxane.

On peut également citer les résines du type triméthylsiloxysilicate commercialisées notamment sous les dénominations X22-4914, X21-5034 et X21-5037 par la société SHIN-ETSU.

Les silicones organomodifiées utilisables conformément à l'invention sont des silicones telles que définies précédemment et comportant dans leur structure un ou plusieurs groupements organofonctionnels fixés par l'intermédiaire d'un groupe hydrocarboné.

Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone-copolyol commercialisé par la société DOW CORNING sous la dénomination DC 1248 ou les huiles SILWET® L 722, L 7500, L 77, L 711 de la société UNION CARBIDE et l'alkyl(C₁₂)-méthicone- copolyol commercialisée par la société DOW CORNING sous la dénomination Q2 5200 ;
- des groupements aminés, substitués ou non, comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C₁-C₄ ;
- des groupements thiols, comme les produits commercialisés sous les dénominations GP 72 A et GP 71 de GENESEE ;
- des groupements alcoxylés, comme le produit commercialisé sous la dénomination SILICONE COPOLYMER F-755 par SWS SILICONES et ABIL WAX® 2428, 2434 et 2440 par la société GOLDSCHMIDT ;
- des groupements hydroxylés, comme les polyorganosiloxanes à fonction hydroxyalkyle décrits dans la demande de brevet français FR-A-85 16334 ;
- des groupements acyloxyalkyle tels que, par exemple, les polyorganosiloxanes décrits dans le brevet US-A-4957732 ;
- des groupements anioniques du type carboxylique comme, par exemple, dans les produits décrits dans le brevet EP 186 507 de la société CHISSO CORPORATION, ou de type alkyl-carboxyliques comme ceux présents dans le produit X-22-3701E de la société SHIN-ETSU ; 2-hydroxyalkylsulfonate ; 2-hydroxyalkylthiosulfate tels que les produits commercialisés par la société GOLDSCHMIDT sous les dénominations ABIL® S201 et ABIL® S255.
- des groupements hydroxyacylamino, comme les polyorganosiloxanes décrits dans la demande EP 342 834. On peut citer, par exemple, le produit Q2-8413 de la société DOW CORNING.

Les silicones telles que décrites ci-dessus peuvent être utilisées seules ou en mélange, en une quantité comprise entre 0,01 et 20 % en poids, de préférence entre 0,1 et 5 % en poids.

Le milieu aqueux cosmétiquement acceptable peut contenir des électrolytes minéraux ou organiques.

Les électrolytes utilisés sont de préférence des sels hydrosolubles minéraux tels que les sels de métaux alcalins, alcalino-terreux, ou d'aluminium d'acide chlorhydrique, sulfurique ou nitrique, ou bien des sels d'acides organiques tels que les carbonates, lactates, citrates ou tartrates de métaux alcalins ou alcalino-terreux ou d'aluminium. Les électrolytes particulièrement préférés sont choisis parmi le sulfate de potassium, le sulfate de sodium, le sulfate de magnésium, le nitrate de calcium, le nitrate de magnésium, le chlorure de sodium, le chlorure de potassium, le carbonate de potassium, le carbonate de sodium et le citrate de sodium.

Ces électrolytes sont présents de préférence dans des proportions allant de 0,1 à 30 % en poids, en particulier de 1 à 10 % en poids, rapporté au poids total de la composition.

Le pH des compositions aqueuses de la présente invention est de préférence fixé à une valeur comprise entre 3 et 11, en particulier entre 5 et 9.

La présente invention a également pour objet un procédé de traitement des matières kératiniques comprenant l'application d'une composition cosmétique selon la présente invention sur les matières kératiniques à traiter.

Elle a également pour objet un procédé de coiffage comprenant l'application d'une composition cosmétique selon la présente invention sur les cheveux, le rinçage des cheveux, puis la mise en forme et le séchage des cheveux rincés.

L'invention est illustrée à l'aide des exemples suivants qui constituent des modes de réalisation préférés des polymères et compositions de la présente invention.

### Exemple 1

On introduit dans un réacteur muni d'un système d'agitation, d'un réfrigérant et d'un thermomètre, 85 g d'acrylate de butyle et 15 g de méthacrylate de diméthylaminoéthyle (MADAME). On dilue avec 100 g de tétrahydrofurane et on ajoute 1 g d'amorceur (Trigonox® 21, commercialisé par la société Akzo). On chauffe pendant 6 heures à reflux du solvant.

L'analyse chromatographique par GPC du polymère ainsi obtenu est effectuée dans le tétrahydrofurane (étalonnage polystyrène) sur un prélèvement de polymère purifié par précipitation dans de l'eau. La masse au maximum du pic d'élution est égale à 80 000.

A la solution précédente on ajoute 200 g de THF, puis on ajoute 1,06 g d'HCl en solution dans 100 g d'eau tout en maintenant une agitation forte au moyen d'un appareil de type Ultra Thurax. Cette quantité d'acide chlorhydrique correspond à celle nécessaire pour neutraliser un tiers des motifs dérivés de méthacrylate de diméthylaminoéthyle, à savoir 0,029 mole pour 100 g de polymère. Après cette étape de neutralisation partielle, on évapore le tétrahydrofurane de manière à obtenir une dispersion aqueuse du polymère ayant une concentration de 20 %. La taille des particules obtenues, déterminée par diffusion de la lumière à l'aide d'un appareil Coulter N4SD, est de 213 nm.

La force de traction maximale (Fₘₐₓ (en N)) enregistrée lors du décollement par traction de deux surfaces circulaires de 0,95 cm², enduites de ce copolymère d'acrylate de butyle et de méthacrylate de diméthylaminoéthyle, est de 6,0 N.

### Exemple 2

On introduit dans un réacteur muni d'un système d'agitation, d'un réfrigérant et d'un thermomètre, 90 g d'acrylate de butyle et 10 g de diméthylaminopropylméthacrylamide (DMAPMA). On dilue avec 100 g de tétrahydrofurane et on ajoute 1 g d'amorceur (Trigonox® 21, commercialisé par la société Akzo). On chauffe pendant 6 heures à reflux du solvant.

L'analyse chromatographique par GPC du polymère ainsi obtenu est effectuée dans le tétrahydrofurane (étalonnage polystyrène) sur un prélèvement de polymère purifié par précipitation dans de l'eau. La masse au maximum du pic d'élution est égale à 80 000.

A la solution précédente on ajoute 200 g de THF, puis on ajoute 2,33 g d'HCl en solution dans 100 g d'eau tout en maintenant une agitation forte de type Ultra Thurax. Cette quantité d'acide chlorhydrique correspond à celle nécessaire pour neutraliser 100 % des motifs dérivés de DMAPMA, à savoir 0,064 mole pour 100 g de polymère. Après cette étape de neutralisation, on évapore le tétrahydrofurane de manière à obtenir une dispersion aqueuse du polymère ayant une concentration de 20 %. La taille moyenne des particules obtenues, déterminée par diffusion de la lumière à l'aide d'un appareil Coulter N4SD est de 18,6 nm.

La force de traction maximale (Fₘₐₓ (en N)) enregistrée lors du décollement par traction de deux surfaces circulaires de 0,95 cm², enduites de ce copolymère d'acrylate de butyle et de diméthylaminopropylméthacrylamide, est de 11,4 N.

### Exemple 3

On prépare les shampooings A et B et l'après-shampooing C suivants :

| | Shampooing A | Shampooing B | Après-shampooing C |
|---|---|---|---|
| lauryléther sulfate de sodium (2,2 OE) à 70 % de matière active | 12,5 % de matière active | 12,5 % de matière active | |
| cocoylbétaïne en solution aqueuse à 30% | 2,5 % de matière active | 2,5 % de matière active | |
| mélange d'alcool cétylstéarylique et d'alcool cétylstéarylique oxyéthyléné (30 OE) | | | 4 % de matière active |
| chlorure de béhényltriméthyl-ammonium à 80 % dans un mélange eau/isopropanol (15/85) | | | 2 % de matière active |
| polymère selon l'exemple 1 | 3 % de matière active | | |
| polymère selon l'exemple 2 | | 3 % de matière active | 3 % de matière active |
| eau | qsp 100 % | qsp 100 % | qsp 100 % |

Les cheveux lavés ou traités avec ces compositions présentent des propriétés cosmétiques intéressantes en termes de volume de corporisation et de facilité de coiffage.

### Exemple 4

On prépare de manière analogue à celle décrite dans l'exemple 1 et 2 un terpolymère cationique à partir de 40 g de méthacrylate de méthoxypolyéthylèneglycol (masse moléculaire 550), de 50 g de diméthylaminopropylméthacrylamide et de 10 g de méthacrylate d'éthoxyéthyle dans 100 g de tétrahydrofurane en présence de 1 g d'amorceur (Trigonox® 21, commercialisé par la société Akzo).

### Exemple 5

On prépare de manière analogue à celle décrite dans l'exemple 1 et 2 un terpolymère cationique à partir de 80 g de d'acrylate de 2-éthylhexyle, 10 g de diméthylaminopropylméthacrylamide (DMAPMA) et de 10 g de méthacrylate d'éthoxyéthyle dans 100 g de tétrahydrofurane en présence de 1 g d'amorceur (Trigonox® 21, commercialisé par la société Akzo).

### Exemple 6

On prépare les shampooings D et E suivants :

| | Shampooing D | Shampooing E |
|---|---|---|
| lauryléther sulfate de sodium (2,2 OE) à 70 % de matière active | 12,5 % de matière active | 12,5 % de matière active |
| cocoylbétaïne en solution aqueuse à 30 % | 2,5 % de matière active | 2,5 % de matière active |
| Merquat® 100 (Nalco) | 0,1 % de matière active | 0,1 % de matière active |
| terpolymère selon l'exemple 4 | 3 % de matière active | |
| terpolymère selon l'exemple 5 | | 3 % de matière active |
| eau | qsp 100 % | qsp 100 % |

Les cheveux lavés ou traités avec ces shampooings présentent des propriétés cosmétiques intéressantes en termes de volume de corporisation et de facilité de coiffage.

## Revendications

1. Polymères radicalaires cationiques ou amphotères, comprenant un
ou plusieurs motifs dérivés de monomères éthyléniques, cationiques ou amphotères, comportant au moins une fonction amine tertiaire ou quaternaire, **caractérisés par le fait qu'**ils présentent une auto-adhésivité, exprimée par la force de traction maximale (Fₘₐₓ (en N)), supérieure ou égale à 2 N et **par le fait qu'**ils comprennent un ou plusieurs motifs dérivés de monomères choisis parmi ceux de formule (la), (Ib), (Ic), (Id) et (le) 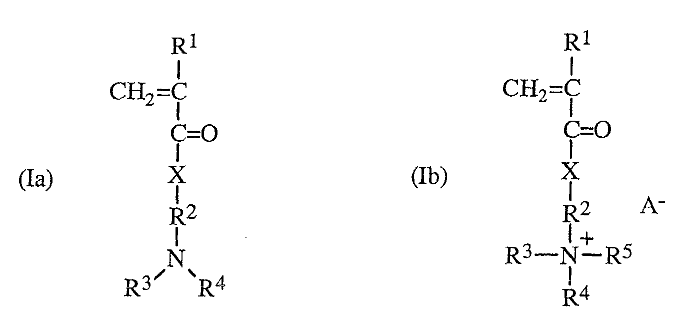 dans lesquelles
R₁ représente un atome d'hydrogène ou un groupe méthyle,
R₂ représente un groupe hydrocarboné divalent en C₁₋₃₀, linéaire, ramifié, cycloaliphatique ou aromatique, pouvant contenir un ou plusieurs hétéroatomes choisis parmi O, N et P,
R₃, R4 et R₅ représentent chacun indépendamment un groupe hydrocarboné en C₁₋₃₀, linéaire, ramifié, cycloaliphatique ou aromatique, pouvant contenir un ou plusieurs hétéroatomes choisis parmi O, N et P,
X représente un atome d'oxygène ou un groupe NH,
A⁻ représente le contre-ion de l'amine quaternaire, choisi de préférence parmi les ions halogénures, sulfate, phosphate et carboxylate tel qu'acétate, 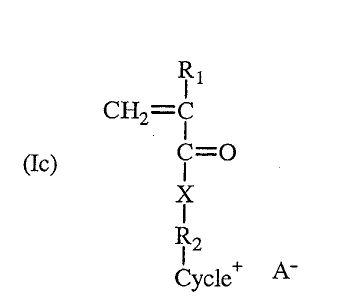
dans laquelle
X, R₁ et R₂ ont la signification indiquée à propos des formules (Ia) et (Ib), et
Cycle⁺ représente un système monocyclique ou bicyclique condensé, cycloaliphatique ou aromatique, comportant une fonction amine tertiaire ou quaternaire, et pouvant renfermer un ou plusieurs hétéroatomes supplémentaires choisis parmi O, N ou P ; 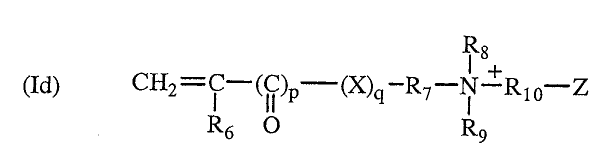 dans laquelle
R₆ représente un atome d'hydrogène ou un groupe alkyle en C₁₋₄ linéaire ou ramifié,
R₈ et R₉ représentent chacun indépendamment un atome d'hydrogène ou un groupe alkyle en C₁₋₄ linéaire ou ramifié, portant éventuellement un groupe COO⁻, SO₃⁻ ou PO₃H⁻,
R₇ et R₁₀ représentent chacun indépendamment un groupe hydrocarboné divalent, en particulier un groupe -(CH₂)ₙ avec n compris entre 1 et 4 inclus, et éventuellement interrompu par un atome d'oxygène,
X est un atome d'oxygène ou un groupe NH,
p et q valent 0 ou 1
Z représente un groupe COO⁻, SO₃⁻ ou PO₃H⁻,
où R₇ peut former avec R₈, R₉ ou X, lorsque ce dernier représente un groupe NH, un hétérocycle à 5, 6 ou 7 chaînons, aromatique ou non-aromatique ; 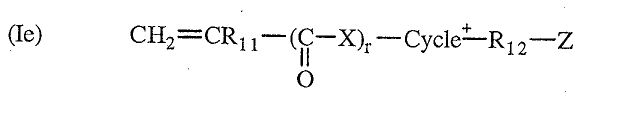
dans laquelle
R₁₁ représente un atome d'hydrogène ou un groupe méthyle,
R₁₂ représente un groupe hydrocarboné divalent en C₁₋₄,
X représente un atome d'oxygène ou un groupe NH,
r vaut 0 ou 1,
Cycle⁺ représente un système monocyclique ou bicyclique condensé, cycloaliphatique ou aromatique, comportant une fonction amine tertiaire ou quaternaire, et pouvant renfermer un ou plusieurs hétéroatomes supplémentaires choisis parmi O, N ou P, et
Z représente un groupe COO⁻, SO₃⁻ ou PO₃H⁻..

2. Polymères selon la revendication 1, **caractérisés par le fait que** Fₘₐₓ est compris entre 2 N et 100 N, de préférence entre 5 et 100 N.

3. Polymères selon la revendication 1 ou 2, **caractérisés par le fait qu'**ils présentent une température de transition vitreuse (Tg), déterminée par analyse calorimétrique différentielle, inférieure à 20 °C, de préférence inférieure à 0 °C et en particulier inférieure à -20 °C.

4. Polymères selon l'une quelconque des revendications précédentes, **caractérisés par le fait que** les monomères éthyléniques cationiques de formule (la) à (Ic) comportant au moins une fonction amine tertiaire sont choisis parmi le (méth)acrylate de diméthylaminoéthyle, le (méth)acrylate de diéthylaminoéthyle, le (méth)acrylate de diméthylaminopropyle, le diméthylaminoéthyl(méth)acrylamide, le diméthylaminopropyl(méth)acryl-amide, le (méth)acrylate de N-morpholinoéthyle, le chlorure de (méth)acrylate de triméthylammonio-éthyle, le chlorure de (méth)acrylate de triméthylammoniopropyle, le chlorure de triméthylammonio-éthyl(méth)acrylamide, le chlorure de triméthylammoniopropyl-(méth)acrylamide et le chlorure de (méth)acrylate de diméthylbenzylammonioéthyle.

5. Polymères selon la revendication 4, **caractérisés par le fait que** les monomères éthyléniques cationiques sont choisis parmi le méthacrylate de diméthylaminoéthyle (MADAME) et le diméthylaminopropylméthacrylamide (DMAPMA)

6. Polymères selon l'une quelconque des revendications précédentes, **caractérisés par le fait que** les monomères amphotères de formule (Id) et (Ie) sont choisis parmi l'hydroxyde de 1-vinyl-2-(3-sulfopropyl)imidazolium, le 1-vinyl-3-(3-sulfopropyl)imidazolium, l'hydroxyde de 1-vinyl-3-(4-sulfobutyl)imidazolium, l'hydroxyde de 1-vinyl-2-méthyl-3-(4-sulfobutyl)imidazolium, l'hydroxyde de 2-vinyl-1-(3-sulfopropyl)pyridinium, l'hydroxyde de 2-méthyl-5-vinyl-1-(3-sulfopropyl)-pyridinium, l'hydroxyde de 4-vinyl-1-(3-sulfopropyl)pyridinium, l'hydroxyde de diméthyl-(2-méthacryloxyéthyl)-3-sulfopropyl)ammonium, l'hydroxyde de diéthyl-(2-méthacryloxyéthoxy-2-éthyl-(3-sulfopropyl)-ammonium, le 4-vinyl-4-(sulfobutyl)pyridinium, l'hydroxyde de N-(3-sulfopropyl)-N-méthacrylamidopropyl-N,N-diméthylammonium, l'hydroxyde de N,N-diméthyl-N-(3-(méthacrylamido)-propyl)-3-(sulfopropyl)ammonium, l'hydroxyde de N,N-diméthyl-N-(3-méthacrylamidopropyl)-N-(3-carboxypropyl)ammonium et l'hydroxyde de N,N-diméthyl-N-(2-méthacryloxyéthyl-N-(3-carboxypropyl)ammonium.

7. Polymères selon l'une quelconque des revendications précédentes, **caractérisés par le fait qu'**ils comprennent en outre des motifs dérivés de monomères éthyléniques non-ioniques.

8. Polymères selon la revendication 7, **caractérisés par le fait que** lesdits monomères éthyléniques non-ioniques sont choisis parmi les monomères non-ioniques qui donnent, par polymérisation, des homopolymères ayant une température de transition vitreuse (Tg) inférieure à 0 °C.

9. Polymères selon la revendication 7 ou 8, **caractérisé par le fait que** les monomères éthyléniques non-ioniques sont choisis parmi les esters de vinyle ou d'allyle, (méth)acrylates et (méth)acrylamides de formule : 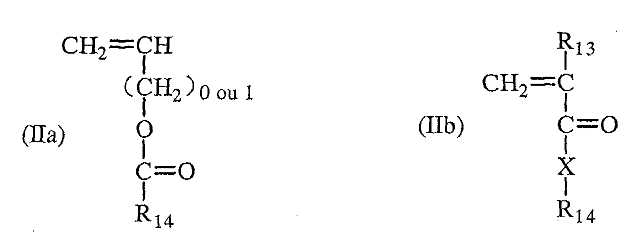 dans laquelle
R₁₃ représente un atome d'hydrogène ou un groupe méthyle,
X représente un atome d'oxygène ou un groupe NH, et
R₁₄ représente un groupe hydrocarboné en C₂₋₆₀, linéaire, ramifié, cycloaliphatique ou aromatique, pouvant contenir un ou plusieurs atomes choisis parmi O, S et P, et notamment une chaîne alcoxy-polyéthylèneglycol.

10. Polymères selon la revendication 9, **caractérisés par le fait que** les monomères éthyléniques non-ioniques sont choisis parmi l'acrylate d'éthyle, de n-butyle, de n-hexyle, de 2-éthylhexyle, de n-nonyle, de lauryle, de n-octadécyle, d'iso-octyle, d'isodécyle, d'hydroxyéthyle, d'hydroxypropyle et de méthoxyéthyle, le méthacrylate de n-hexyle, de 2-éthylhexyle, d'éthoxyéthyle, d'isodécyle, de méthoxyéthyle, d'alcoxy en C₁₋₃₀-PEG (avec 5 à 30 motifs d'oxyde d'éthylène), le propionate de vinyle, et les néoalcanoates de vinyle tels que le néononanoate de vinyle et le néododécanoate de vinyle.

11. Polymères selon la revendication 10, **caractérisés par le fait que** les monomères éthyléniques non-ioniques sont choisis parmi l'acrylate de n-butyle, le méthacrylate d'éthoxyéthyle, l'acrylate de 2-éthylhexyle et le méthacrylate de méthoxy-PEG (avec 5 à 30 motifs d'oxyde d'éthylène).

12. Polymères selon la revendication 7, **caractérisés par le fait que** lesdits monomères éthyléniques non-ioniques sont choisis parmi les monomères vinyliques à chaîne latérale siliconée, le chlorotrifluoroéthylène, le tétrafluoroéthylène, et les monomères vinyliques, allyliques ou (méth)acryliques à chaîne latérale hydrocarbonée perhalogénée, en particulier perfluorée, tels que le (méth)acrylate de perfluorohexyle ou le (méth)acrylate de perfluorooctyle.

13. Polymères radicalaires cationiques ou amphotères selon l'une quelconque des revendications 8 à 12, **caractérisés par le fait que** les motifs dérivés de monomères éthyléniques cationiques ou amphotères, représentent de 1 à 50 % en poids du polymère, de préférence de 1 à 20 % en poids du polymère, et les motifs dérivés de monomères éthyléniques non-ioniques donnant des homopolymères à Tg < 0 °C représentent de 50 % à 99 % en poids du polymère, de préférence de 80 à 99 % en poids du polymère.

14. Polymères selon l'une quelconque des revendications précédentes, **caractérisés par le fait qu'**ils comprennent en outre des motifs dérivés de monomères éthyléniques anioniques.

15. Polymères selon la revendication 14, **caractérisés par le fait que** lesdits monomères éthyléniques anioniques sont choisis parmi l'acide acrylique, l'acide méthacrylique, l'acide itaconique, l'acide crotonique, l'acide fumarique, l'acide maléique, l'acide vinylbenzoïque, l'acide vinylbenzènesulfonique, l'acide acrylamidopropanesulfonique et l'acide vinylphosphonique ou les sels d'addition de bases minérales ou organiques de ces acides.

16. Polymères selon l'une quelconque des revendications précédentes **caractérisés par le fait qu'**ils sont choisis parmi
• les copolymères d'acrylate de butyle et de méthacrylate de diméthylaminoéthyle,
• les copolymères d'acrylate de butyle et de diméthylaminopropylméthacrylamide,
• les terpolymères de méthacrylate méthoxy-PEG(avec de 5 à 30 motifs OE), de diméthylaminopropylméthacrylamide et de méthacrylate d'éthoxyéthyle, et
• les terpolymères d'acrylate de 2-éthylhexyle, de diméthylaminopropylméthacrylamide et de méthacrylate d'éthoxyéthyle.

17. Composition cosmétique contenant, dans un milieu cosmétiquement acceptable, au moins un polymère cationique ou amphotère auto-adhésif selon l'une quelconque des revendications précédentes.

18. Composition cosmétique selon la revendication 17, **caractérisée par le fait qu'**elle contient de 0,01 % à 40 %, de préférence de 0,05 à 20 % et en particulier de 0,1 à 10 % en poids d'au moins un polymère cationique ou amphotère auto-adhésif.

19. Composition cosmétique selon la revendication 17 ou 18, **caractérisée par le fait qu'**il s'agit d'une composition de soin, de maquillage ou de fixation des matières kératiniques humaines, en particulier des cheveux et des cils.

20. Composition cosmétique selon la revendication 19, **caractérisée par le fait qu'**il s'agit d'une composition de coiffage.

21. Composition cosmétique selon la revendication 20, **caractérisée par le fait qu'**il s'agit d'une composition de coiffage à rincer.

22. Composition cosmétique selon l'une des revendications 17 à 21, **caractérisée par le fait qu'**elle contient en outre des additifs choisis parmi les agents tensioactifs, des polymères anioniques, amphotères, zwitterioniques ou non ioniques, des polymères cationiques différents des polymères cationiques autoadhésifs selon l'une des revendications 1 à 16, des agents nacrants et/ou opacifiants, des solvants organiques, des parfums, des huiles minérales, végétales et/ou synthétiques, des esters d'acides gras, des pigments et colorants, des silicones, des particules minérales ou organiques, des agents de stabilisation du pH, des agents conservateurs et des agents absorbant les UV.

23. Composition cosmétique selon la revendication 22, **caractérisée par le fait que** les polymères cationiques sont choisis parmi les homopolymères de sel de diallyldiméthylammonium et les copolymères de sel de diallyldiméthylammonium et d'acrylamide

24. Composition cosmétique selon la revendication 22, **caractérisée par le fait que** les agents tensioactifs sont choisis parmi les agents tensioactifs anioniques, non ioniques, amphotères et cationiques et des mélanges de ceux-ci.

25. Composition cosmétique selon la revendication 24, **caractérisée par le fait que** les agents tensioactifs anioniques sont présents à raison de 0,5 à 60 % en poids, de préférence de 5 à 20 % en poids, et que les agents tensioactifs non-ioniques, amphotères et cationiques sont présents à raison de 0,1 % à 30 % en poids, de préférence de 0,5 % à 25 % en poids par rapport au poids total de la composition.

26. Composition cosmétique selon la revendication 22, **caractérisée par le fait que** les silicones sont choisies parmi les silicones volatiles ou non volatiles, cycliques, linéaires ou ramifiées, modifiées ou non par des groupements organiques

27. Composition cosmétique selon la revendication 26, **caractérisée par le fait que** les silicones sont présentes à raison de 0,01 à 20 % en poids, de préférence de 0,1 à 5 % en poids.

28. Utilisation en cosmétique des polymères radicalaires cationiques ou amphotères autoadhésifs selon l'une quelconque des revendications 1 à 16.

29. Procédé de traitement des matières kératiniques comprenant l'application d'une composition cosmétique selon l'une quelconque des revendications 17 à 27 sur les matières kératiniques à traiter.

30. Procédé de coiffage comprenant l'application d'une composition cosmétique selon l'une quelconque des revendications 17 à 27 sur les cheveux, le rinçage des cheveux, puis la mise en forme et le séchage des cheveux rincés.
